Europäisches Patentamt

European Patent Office

Office européen des brevets

(11) Veröffentlichungsnummer: **0 513 614 A1**

## EUROPÄISCHE PATENTANMELDUNG

(21) Anmeldenummer: **92107511.5**

(22) Anmeldetag: **04.05.92**

(51) Int. Cl.5: **A61C 19/02**, A61B 19/02, A61L 2/26

(30) Priorität: **11.05.91 DE 9105867 U**

(43) Veröffentlichungstag der Anmeldung:
**19.11.92 Patentblatt 92/47**

(84) Benannte Vertragsstaaten:
**AT BE CH DE DK ES FR GB GR IT LI LU NL PT SE**

(71) Anmelder: **Ulschmid, Werner**
**Schwalbenstrasse 4b**
**W-8800 Ansbach(DE)**

(72) Erfinder: **Ulschmid, Werner**
**Schwalbenstrasse 4b**
**W-8800 Ansbach(DE)**

(74) Vertreter: **Gallo, Wolfgang, Dipl.-Ing. (FH) et al**
**Patentanwälte Dipl.-Ing. L. Fleuchaus,**
**Dipl.-Phys. H. Schroeter, Dipl.-Ing K.**
**Lehmann, Dipl.-Ing.W. Wehser, Dipl.-Ing.(FH)**
**W. Gallo, Ludwigstrasse 26**
**W-8900 Augsburg(DE)**

(54) **Sterilisierfähiges Behältnis für einzelne medizinische und chirurgische Bestecke, Instrumente und Geräte.**

(57) Behältnis zur Aufnahme einzelner medizinischer oder chirurgischer Bestecke, Instrumente oder Geräte zum Zwecke des Sterilisierens und anschließenden sterilen Aufbewahrens in Form einer mindestens teilweise durchsichtigen, mehrfach sterilisierfähigen steifen Box (10, 20) aus sterilisiertemperaturbeständigem Kunststoff mit einem Unterteil (10) und einem damit zusammenwirkenden und keimdicht abschließenden Deckel (20), die mittels einer keimdichten, jedoch für Sterilisierdampf bzw. Sterilisiergas durchlässigen Filterschicht (30) abgedeckte Wanddurchbrüche (11) aufweist.

FIG. 3

Rank Xerox (UK) Business Services

Die Erfindung betrifft ein sterilisierfähiges, mindestens teilweise durchsichtiges Behältnis zur Aufnahme einzelner medizinischer und chirurgischer Bestecke, Instrumente oder Geräte zum Zwecke des Sterilisierens und anschließenden sterilen Aufbewahrens.

Neben der Bereithaltung ganzer Operationsbestecksätze oder dgl. in sterilem Zustand ist es in der medizinischen und chirurgischen Praxis in großem Umfang notwendig, auch einzelne medizinische Gerätschaften wie beispielsweise Scheren, Spritzen oder dgl. jeweils einzeln steril verpackt aufzubewahren, so daß sie jederzeit einzeln oder Beeinträchtigung der Sterilität anderer Instrumente oder Geräte benutzbar sind. Als Behältnis für solche einzelnen medizinischen bzw. chirurgischen Bestecke, Instrumente und andere Geräte dienen bekanntermaßen aus durchsichtigen Einschweißfolien gefertigte Beutel, die jedoch nur einmal verwendbar sind und nach einmaligem Gebrauch weggeworfen werden. Daher sind die Einschweißfolienbeutel verhältnismäßig teuer. Sie sind auch verhältnismäßig umständlich zu handhaben. Darüberhinaus besteht bei den Einschweißfolienbeuteln das Problem, daß die Folien in sich instabil sind und nur verhältnismäßig kurze Zeit Sterilität gewährleisten und im übrigen gegen mechanische Beschädigungen sowohl von außen als auch von innen empfindlich sind, beispielsweise durch die Spitzen verpackter Scheren oder dgl. leicht durchstoßen werden können. Schließlich schaffen die großen Mengen geöffneter und damit verbrauchter Folienbeutel in einem Krankenhaus große Mengen anfallenden Folienmülls.

Der Erfindung liegt die Aufgabe zugrunde, dem Problem des bei den herkömmlichen, als Folienbeutel ausgebildeten Behältnissen unvermeidbar großen Verbrauchs an Kunststoffolien und des unvermeidbar großen Anfalls an Folienmüll abzuhelfen.

Diese Aufgabe wird gemäß der Erfindung durch die im Anspruch 1 angegebene Ausbildung des Behältnisses gelöst.

Vorteilhafte Ausgestaltungen der Erfindung sind Gegenstand der Unteransprüche.

Das erfindungsgemäße Behältnis hat den Vorteil vielmaliger Verwendbarkeit über einen längeren Zeitraum hinweg, d.h. er kann immer wieder neu zum Sterilisieren und zum sterilen Aufbewahren eines Bestecks oder Geräts verwendet werden. Damit fällt praktisch überhaupt kein Müll an, und außerdem ergibt sich eine beträchtliche Kosteneinsparung, denn trotz des gegenüber einem Einschweißfolienbeutel mehrfach höheren Preises des erfindungsgemäßen steifen Behältnisses amortisieren sich die Kosten nach einer relativ geringen Anzahl von Benutzungszyklen, und bei weiteren vielfachen Benutzungszyklen ergeben sich dann echte Kosteneinsparungen. Bei Wahl eines entsprechenden Kunststoffes ist auch, wenn das Behältnis nach langfristigem Gebrauch aus der Benutzung herausgenommen wird, ein vollständiges Recycling möglich.

Das erfindungsgemäße Behältnis kann, wie in den Unteransprüchen näher angegeben, stapelbar bzw. in Paketen anzuordnen sein, um eine Vielzahl solcher Behältnisse mit Inhalt gemeinsam zu sterilisieren, ohne daß der Zutritt des Sterilisierdampfes in jedes einzelne Behältnis behindert wird, was durch entsprechende Formgebung und Sicherstellung von verbleibenden Strömungszwischenräumen zwischen zusammengepackten Behältnissen erreicht wird.

Steife, mehrfach benutzbare Behältnisse für einzelne Bestecke, Instrumente oder Geräte, wie sie nach der Erfindung vorgesehen sind, gibt es bislang nicht. Bekannt sind lediglich große Sterilisierbehälter zum gemeinsamen Sterilisieren und Aufbewahren ganzer Operationsbestecksätze oder ganzer Serien von Zahnarztbohrern. Derartige bekannte große Sterilisierbehälter für ganze Operationsbestecke, wie sie beispielsweise aus der DE-OS 29 30 324 oder der DE-OS 35 44 645 bekannt sind, haben einen außerordentlich komplizierten Aufbau, der für kleine Behältnisse zum Sterilisieren und Aufbewahren von Einzelgerätschaften unbrauchbar ist.

Bei einem im Gbm 87 11 391 beschriebenen großen Sterilisierbehälter in Form eines metallenen wannenförmigen Kastenunterteils und eines darauf festspannbaren Deckels sind im Deckel Perforationsbereiche vorgesehen, die mit Filterschichten abgedeckt sind, um den Sterilisierdampf durchtreten zu lassen, im übrigen aber keimdicht abzuschließen. Auch diese aus dieser Druckschrift bekannte Konstruktion ist natürlich auf Kleinbehälter nicht übertragbar.

Die Erfindung schafft damit erstmals die Möglichkeit, statt aufwendiger Einschweißfolienverpakkungen feste und dauerhafte, vielfach verwendbare Kleinbehältnisse für Einzelgerätschaften vorzusehen, die überdies nicht nur zum Sterilisieren und Aufbewahren von Bestecken, Instrumenten und Geräten innerhalb von Krankenhäusern, sondern auch als wiederverwendbare Transportverpackungen geeignet sind.

Einige Ausführungsbeispiele der Erfindung werden nachstehend unter Bezugnahme auf die anliegenden Zeichnungen beispielsweise mehr im einzelnen beschrieben. In den Zeichnungen zeigt:

Fig. 1    in perspektivischer Darstellung und auseinandergezogen ein Behältnis nach der Erfindung in Form einer flachen Box mit angelenktem Deckel in geöffnetem Zustand,

Fig. 2    einen vergrößerten Querschnitt durch das in Fig. 1 dargestellte Behältnis,

Fig. 3 in auseinandergezogener perspektivischer Darstellung ein Behältnis nach der Erfindung, wiederum in Form einer flachen Box, jedoch mit aufsetzbarem Deckel,

Fig. 4 einen abgebrochenen Querschnitt durch das Behältnis nach Fig. 3,

Fig. 5 einen Querschnitt durch eine weitere Ausführungsform eines Behältnisses nach der Erfindung, das die Form einer Box haben kann,

Fig. 6 einen Querschnitt durch eine noch weitere Ausführungsform eines boxförmigen Behältnisses nach der Erfindung,

Fig. 7 im Axialschnitt ein zylindrisches Behältnis nach der Erfindung, und

Fig. 8 nochmals im Querschnitt eine Ausführungsform eines boxförmigen Behältnisses nach der Erfindung mit im Deckel gebildeten Durchbrüchen.

Vorweg ist zu den Zeichnungen zu bemerken, daß die Schnittdarstellungen alle vergrößert dargestellt und nicht unbedingt maßstäblich sind, was die Wandstärken betrifft, vielmehr ist die Darstellung im Hinblick auf eine möglichst klare Erläuterung der wesentlichen Merkmale gestaltet.

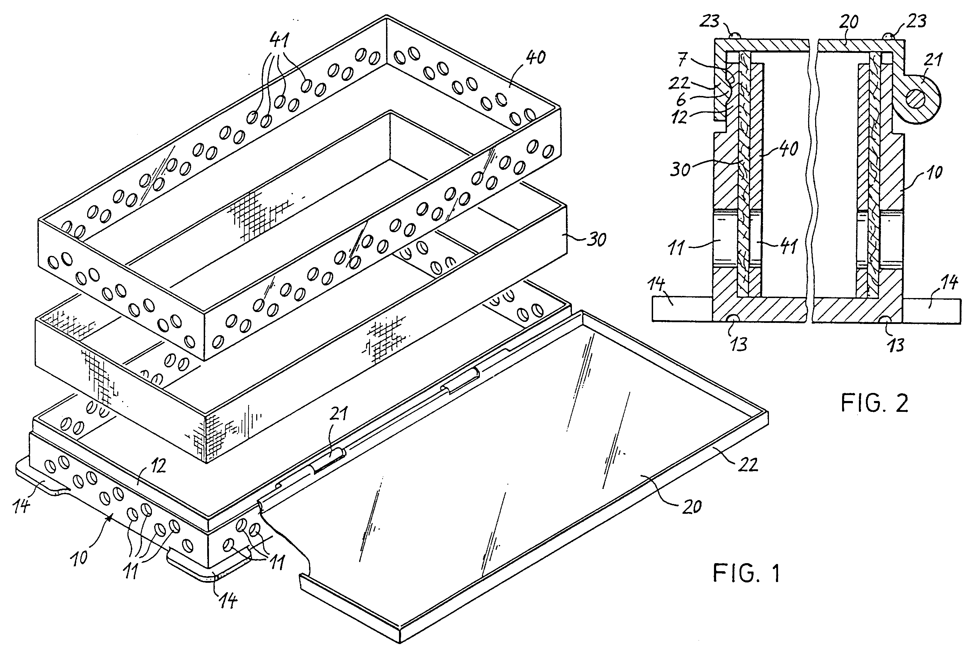

Das in den Fig. 1 und 2 dargestellte Behältnis in Form einer flachen Box besteht aus einem Unterteil 10, einem Deckel 20, der über Scharniere 21 am Unterteil 10 angelenkt ist, eine Filterschicht 30 und einem Einsatz 40.

Die Filterschicht 30 kann, wie dargestellt, durch ein geschlossenes Band gebildet sein, statt dessen können aber auch vier gesonderte Streifen verwendet werden. Die Filterschicht besteht vorzugsweise aus einem dichten Gewebe mit einer Porengröße von nicht mehr als 0,5 μm. Wie Fig. 2 zeigt, dient der Einsatz 40 dazu, die Filterschicht 30 zwischen sich und den Innenwandflächen des Unterteils 10 zu halten.

Die Seitenwände des Unterteils 10 und der Einsatz 40 sind mit sich deckenden Druchbrüchen 11 bzw. 41 versehen, die in ihrer Gesamtfläche mindestens 10% der Bodenfläche des Unterteils ausmachen sollten, so daß ein ausreichender Durchtritt von Sterilisierdampf gewährleistet werden kann.

Der Deckel 20 ist mit einer umlaufenden Randleiste 22 versehen, welche bei geschlossenem Deckel gemäß Fig. 2 den oberen Randteil 12 der Seitenwände des Unterteils klemmend übergreift, um einen keimdichten Verschluß herzustellen. Ein mit einer komplementären Vertiefung 7 des Unterteil-Randteils 12 zusammenwirkender Noppenwulst 6 an der Innenseite der Deckelrandleiste 22 bewirkt ein Einrasten des Deckels in seiner Schließstellung auf dem Unterteil.

Wie Fig. 2 zeigt, ist die Filterschicht 30 so bemessen, daß sie mit ihren oberen Randkanten an der Innenfläche des geschlossenen Deckels anliegt.

Um mehrere solche Behältnisse zum Zwecke des gemeinsamen Sterilisierens übereinander stapeln zu können, ohne daß die Gefahr des gegenseitigen Verrutschens und damit möglicherweise des Umkippens des Stapels besteht, sind an der Deckeloberseite Positioniernoppen 23 und an der Bodenunterseite des Unterteils 10 an den entsprechenden Positionen komplementäre Vertiefungen 13 gebildet, die beim Aufeinanderstapeln mehrerer Behältnisse jeweils ineinandergreifen und die gestapelten Behältnisse verschiebungs- und verrutschfrei vertikal übereinander positionieren.

Außerdem sind am Unterteil im Bereich der Ecken als Abstandhalter dienende seitliche Vorsprünge 14 gebildet, welche die Aufgabe haben, bei mehreren zum gemeinsamen Sterilisieren nebeneinander angeordneten Stapeln von solchen Behältnissen diese mit gegenseitigem seitlichem Abstand zu halten, damit Sterilisierdampf ungehindert zu den seitlichen Durchbrüchen jedes einzelnen Behältnisses gelangen kann.

Gemäß Fig. 1 sind die Durchbrüche 11 bzw. 41 an allen vier Seiten des Unterteils 10 bzw. des Einsatzes 40 vorgesehen. Zur Aufnahme länglicher spitzer Gegenstände wie beispielsweise Scheren kann es aber zweckmäßig sein, die Durchbrüche nur an den Längsseiten des Verhältnisses vorzusehen und die beiden Schmalseiten ohne Durchbrüche zu belassen, damit nicht die Gefahr eines unerwünschten Durchstoßens der Filterschicht im Bereich eines stirnseitigen Durchbruchs an einer der Schmalseiten des Behältnisses besteht. In diesem Fall ist es auch nicht notwendig, daß die als Abstandshalter dienenden Vorsprünge 14, wie in Fig. 1 dargestellt, über Eck verlaufen, vielmehr können sie dann auf die Längsseiten beschränkt sein.

Damit der Inhalt eines geschlossenen und sterilisierten Behältnisses sichtbar ist, ist mindestens der Deckel 20 oder auch das ganze Behältnis aus einem transparenten, vorzugsweise glasklaren Kunststoff hergestellt. Es versteht sich von selbst, daß ein Kunststoff auszuwählen ist, der den beim Sterilisieren erforderlichen Temperaturen standhält. Außerdem sollte dieser Kunststoff auch noch recyclingfähig sein.

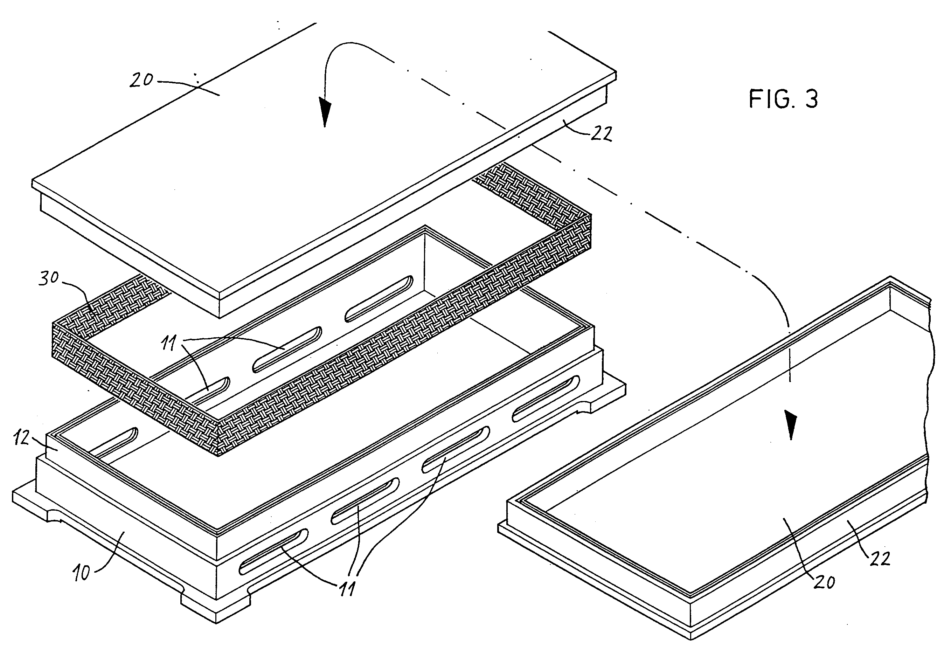

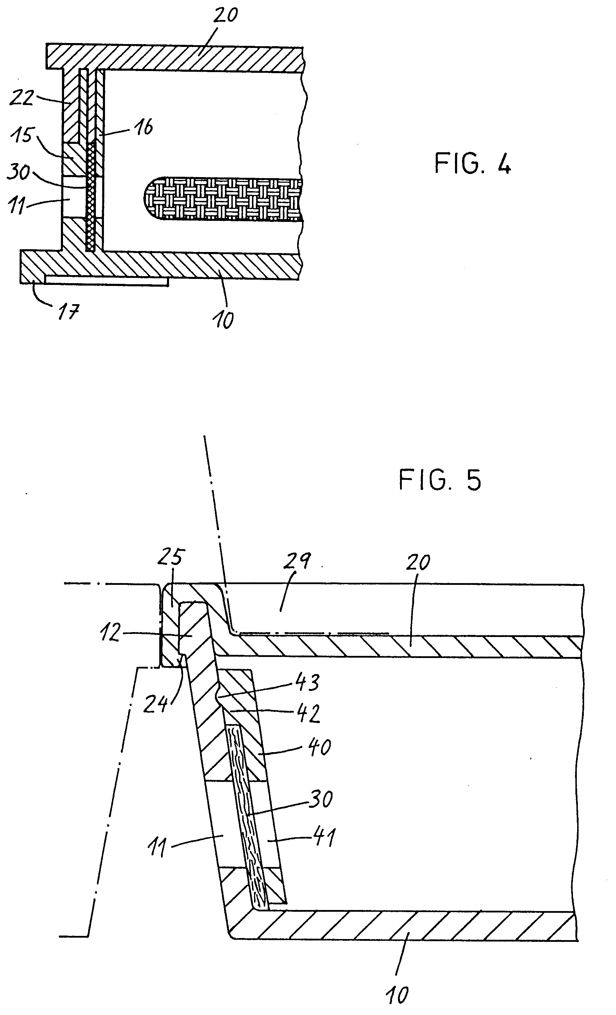

Die in den Fig. 3 und 4 gezeigte Ausführungsform eines Behältnisses nach der Erfindung ist wiederum als flache Box ausgebildet und besteht aus einem Unterteil 10, einem Deckel 20 und einer Filterschicht 30.

Der Deckel 20 ist hier nicht angelenkt, sondern als loses, auf das Unterteil aufsetzbares Teil ausgebildet.

Die Seitenwände des Unterteils 10 sind längsgeschlitzt, wobei der zwischen dem äußeren Wandsteg 15 und den inneren Wandsteg 16 gebildete Schlitz gemäß Fig. 4 zur Aufnahme der Filterschicht 30 dient. Natürlich ist aber alternativ dazu auch eine Konstruktion entsprechend Fig. 1 möglich, nämlich der Gestalt, daß der innere Wandsteg 16 durch einen losen Einsatz nach Maßgabe der Fig. 1 ersetzt ist.

Der Deckel 20 ist wiederum mit einer umlaufenden Randleiste 22 versehen, die gemäß Fig. 4 ebenso wie die Seitenwände des Unterteils geschlitzt sind, so daß die Randleiste des Deckels und der obere Randteil der Seitenwände des Unterteils mehrfach verzahnt ineinandergreifend miteinander zusammenwirken. Dadurch erhält man eine labyrinthartige, einen keimdichten Verschluß gewährleistende Dichtungsfuge.

Statt kleiner runder Durchbrüche wie beim Ausführungsbeispiel nach den Fig. 1 und 2 sind hier längliche größere Durchbrüche 11 in den Seitenwänden des Unterteils vorgesehen. Auch hier gilt wiederum, daß diese Durchbrüche, wie dargestellt, vorzugsweise auf die Längsseiten des Behältnisses beschränkt sind, um ein ungewolltes Durchstoßen der Filterschicht durch einen in dem Behältnis untergebrachten spitzen Gegenstand an den Schmalseiten des Unterteils zu vermeiden. Trotzdem können natürlich gewünschtenfalls auch die Schmalseiten mit Durchbrüchen versehen sein.

Als Positionierhilfe beim Stapeln ist bei diesem Ausführungsbeispiel statt der Noppen und Vertiefungen, wie sie aus Fig. 2 ersichtlich sind, jeweils ein nach unten vorspringender Rand 17 der Abstandhaltevorsprünge 14 vorgesehen, der beim Stapeln die Ecken des Deckels eines darunterliegenden Behältnisses umgreift.

Fig. 5 zeigt in Form eines abgebrochenen Querschnitts ähnlich Fig. 4 eine weitere Ausführungsform eines Behältnisses nach der Erfindung, das wie die Ausführungsbeispiele nach den Fig. 1 bis 4 als flache Box ausgebildet ist, wobei natürlich diese flache Box nicht unbedingt die aus den Fig. 1 bzw. 3 ersichtliche Rechteckform zu haben braucht, sondern auch länglicher und schmäler, keilförmig, mit abgerundeten Ecken oder Stirnenden, oval oder in jeder anderen geeigneten Form gestaltet sein kann.

Bei der Ausführungsform nach Fig. 5 besteht das Behältnis wiederum aus einem Unterteil 10 in Form einer flachen Schale, einem darauf aufsetzbaren Deckel 20, einer Filterschicht 30 und einem losen Einsatz 40.

Eine Besonderheit dieses Ausführungsbeispiels gegenüber den vorbeschriebenen Ausführungsformen besteht darin, daß die Seitenwände des Unterteils 10 nicht rechtwinklig zur Bodenwand verlaufen, sondern konisch, d.h. nach oben etwas schräg nach außen verlaufend angeordnet sind. Die gleiche konische Form haben auch die Filterschicht 30 und der Einsatz 40, der zur Halterung der Filterschicht im Unterteil dient. Aus Fig. 5 ist leicht ersichtlich, daß diese konische Gestaltung der Seitenwände, der Filterschicht und des Einsatzes ein wesentlich leichteres Einsetzen der Filterschicht und des Einsatzes in das Unterteil ermöglicht als bei dem Ausführungsbeispiel nach Fig. 1. Selbstverständlich sind wiederum zumindest an den Längsseiten der Box miteinander fluchtende Wanddurchbrüche 11 bzw. 41 in den Unterteil-Seitenwänden und dem Einsatz 40 vorgesehen.

Wie aus Fig. 5 weiter hervorgeht, erstreckt sich bei dieser Ausführungsform die Filterschicht 30 nicht über die gesamte Höhe des Einsatzes, sondern oben hat der Einsatz eine auswärts vorspringende Randleiste 42, der mit einem auswärts vorspringenden, in eine entsprechende komplementäre Nut an der Innenwandfläche der Unterteilseitenwände eingreifenden Wulst 43 versehen sein kann. Der Wulst 43 und die komplementäre Nut dienen als Rastorgane zum Arretieren des Einsatzes 40 im Unterteil, ermöglichen jedoch dessen Herausnahme, wenn die Filterschicht 30 ausgewechselt werden soll. Statt dessen könnten auch in an sich bekannter Weise Rastnasen an der Innenseite der Unterteilwände gebildet sein, die beim Einsetzen des Einsatzes 40 in das Unterteil über der oberen Randkante der Randleiste 42 einrasten. Damit wird verhindert, daß beim Öffnen des Deckels 20 ungewollt auch der Einsatz samt der Filterschicht herausfallen kann. Außerdem ermöglicht diese Konstruktion das leichte und gleichförmige Einbringen der Filterschicht 30 zwischen den Unterteilseitenwänden und dem Einsatz, indem sie entweder zuerst in das Unterteil eingelegt und dann der Einsatz 40 eingesetzt wird, oder indem die Filterschicht um den Einsatz herumgelegt und dann mit dem Einsatz zusammen in das Unterteil eingesetzt wird, und bei diesem Einsetzen kann aufgrund der konischen Gestaltung der Unterteilseitenwände und des Einsatzes ein gewisses Zusammentreffen der Filterschicht mit dem Ziel der satten Anlage sowohl an den Unterteilseitenwänden als auch am Einsatz erreicht werden, wobei die so erreichte Einbaulage durch die genannten Rastorgane gesichert wird.

Der Deckel 20 kann, wie in Fig. 5 gezeigt, einen hochgewölbten und umgefalzten Randwulst 25 haben, der die Randleiste 22 bei den vorbeschriebenen Ausführungsformen ersetzt und den hochstehenden oberen Randteil 12 des Unterteils umgreift. Auch zwischen den Randwulst 25 und dem Randteil 12 des Unterteils können miteinander zusammenwirkende Rastorgane in Form von elastisch übereinander rastenden Rastleisten vorgesehen sein, um sowohl die Dichtigkeit zu verbessern, als auch den aufgesetzten Deckel auf dem Unterteil zu arretieren. In Fig. 5 ist am unteren Ende des Randwulstes 25 eine einwärts vorspringende Rastleiste 24 vorgesehen, die den zurückgestuften oberen Randteil 12 der Unterteilseitenwände untergreift.

Die Geometrie der Anordnung ist so bemessen, daß die vom Randwulst 25 umschlossene Deckelvertiefung 29 in ihren Abmessungen gerade den Abmessungen des Bodenbereichs des Unterteils entspricht, so daß beim Stapeln, wie strichpunktiert angedeutet, eine aufgesetzte weitere Box gerade in die Deckelvertiefung 29 der darunterliegenden Box eingestellt werden kann. Damit wird sowohl eine verrutsch- und verschiebesichere Positionierung aufeinandergestapelter Boxen erreicht als auch ein Zwangsabstand zwischen nebeneinander aufgestellten Stapeln hergestellt (siehe strichpunktiert angedeutete benachbarte Box), so daß Sterilisierdampf ohne weiteres zwischen benachbarten Stapeln zu den Längsseiten jeder einzelnen Box gelangen kann.

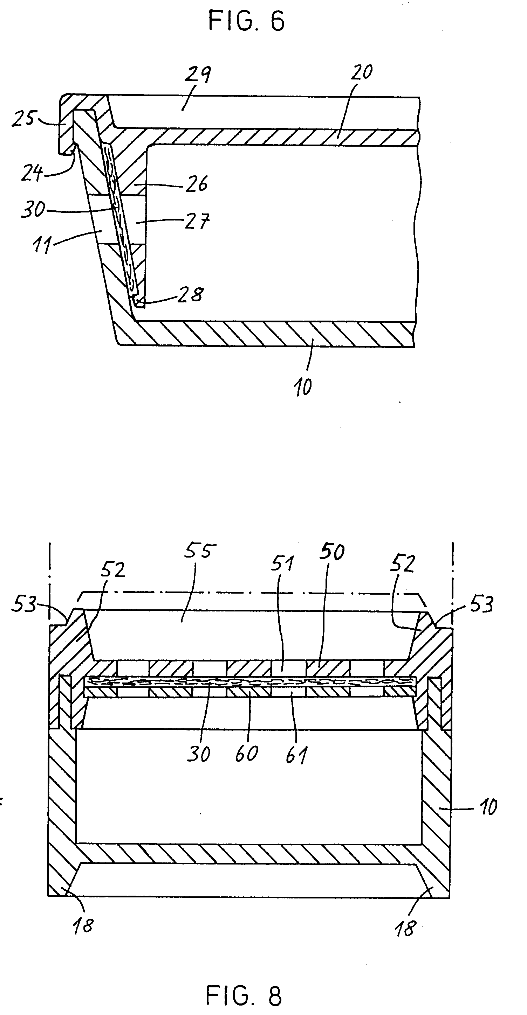

Fig. 6 zeigt eine Ausführungsform, die hinsichtlich der grundsätzlichen Gestaltung von Unterteil 10 und Deckel 20 und im Hinblick auf die Stapelbarkeit unter Gewährleistung seitlicher Zwischenräume weitgehend der Ausführungsform nach Fig. 5 entspricht.

Jedoch hat das Ausführungsbeispiel nach Fig. 6 die Besonderheit, daß der bei der Konstruktion nach Fig. 5 vorgesehene Einsatz durch einen von der Deckelinnenseite nach unten ragenden konischen Steg 26 ersetzt ist, der mit bezüglich den Wanddurchbrüchen 11 des Unterteils 10 deckungsgleichen Durchbrüchen 27 versehen ist. Die Filterschicht 30 kann dauerhaft auf der konischen Außenfläche des Stegs 26 fixiert sein, beispielsweise durch eine auswärts vorspringende Nase 28 am unteren Stegende, die ein Herunterrutschen der Filterschicht verhindert. Freilich kann die Filterschicht auch durch Verklebung auf dem Steg 26 befestigt oder alternativ dazu an der Innenfläche der Unterteilseitenwände befestigt sein. Allerdings ist zu beachten, daß bei dieser Ausführungsmöglichkeit die Filterschicht eher mechanischen Beschädigungen ausgesetzt ist als bei den vorbeschriebenen Ausführungsbeispielen und bei Fixierung am Deckelsteg oder am Unterteil nicht ohne weiters ausgetauscht werden kann.

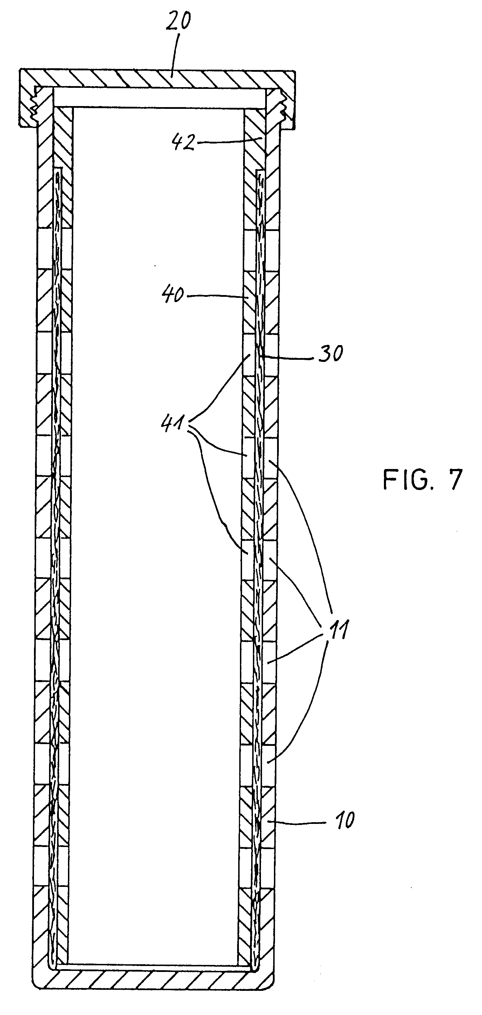

Sodann wird nun unter Bezugnahme auf Fig. 7 zur Abrundung der möglichen Ausführungsbeispiele noch eine zylindrische Ausführungsform eines steifen Behältnisses nach der Erfindung beschrieben. Diese besteht aus einem becherartigen Unterteil 10 mit einem Einsatz 40 in Form einer Hülse und einer zwischen der Umfangswand des Unterteils 10 und der Einsatzhülse 40 eingesetzten schlauchförmigen Filterschicht 30, sowie aus einem Deckel 20, der auf das becherförmige Unterteil 10 aufgesetzt ist. Die Umfangswand des Unterteils 10 und die Einsatzhülse 40 sind mit Wanddurchbrüchen 11 bzw. 41 versehen, die sich im zusammengesetzten Zustand dieser beiden Bauteile mindestens im wesentlichen decken. Das kann dadurch erreicht werden, daß zwischen der Randleiste 42 am oberen Ende der Einsatzhülse 40 und der entsprechenden Innenwandfläche des Unterteils 10 formschlüssig zusammenwirkende Positionierelemente angeordnet sind, welche die relative Drehstellung zwingend vorgeben, zumindest in einem der Winkelteilung der Wanddurchbrüche entsprechenden Winkelraster, oder daß in Unterteil und Hülse jeweils in entsprechenden Axialpositionen in Umfangsrichtung verlaufende längliche Durchbrüche mit solcher Größe und so geringen gegenseitigen Abständen gebildet sind, daß unabhängig von der jeweiligen winkelmäßigen Relativposition von Unterteil und Hülse eine zum Sterilisieren ausreichende Teilüberdeckung der Durchbrüche in jedem Fall gewährleistet ist. Ebenso wie bei dem Ausführungsbeispiel nach Fig. 5 können zwischen der Randleiste 42 der Eingangshülse und dem Unterteil miteinander zusammenwirkende Rastorgane zur axialen Sicherung der Hülse im Unterteil vorgesehen sein.

Der Deckel 20 könnte bei dem Ausführungsbeispiel nach Fig. 6 in ähnlicher Weise wie bei der Ausführungsform nach Fig. 5 ausgebildet sein, nämlich mit einem umgebogenen Randwulst zum Umgreifen des oberen Becherrandes, kann aber auch, wie in Fig. 6 dargestellt, als Schraubverschluß mit einem keimdicht dichtenden Gewinde ausgebildet sein.

Seitliche Abstandhalter sind bei der zylindrischen Form des Behältnisses nicht erforderlich, da beim Zusammenstellen mehrerer solcher Behältnisse nebeneinander zwangsläufig Zwischenräume zwischen diesen verbleiben, die den Zutritt von Sterilisierdampf zu den an den Umfangswänden vorgesehenen Wanddurchbrüchen ermöglichen.

Schließlich ist in Fig. 8 nochmals ein boxförmiges Behältnis nach der Erfindung dargestellt, das sich jedoch von den in den Fig. 1 bis 6 dargestellten Behältnissen dadurch unterscheidet, daß die Wanddurchbrüche nicht in den Seitenwänden des Unterteils, sondern im Deckel 50 als Durchbrüche 51 gebildet sind. Das Unterteil 10 hat dementsprechend geschlossene Wände, und der Deckel 50 trägt innenseitig die Filterschicht 30, die mit Hilfe einer Halteplatte 60 mit zu den Deckeldurchbrüchen 51 deckungsgleich angeordneten Durchbrüchen 61 an der Deckelinnenseite fixiert ist.

Damit bei gestapelten Boxen der Sterilisierdampf ungehindert zu den Deckeldurchbrüchen 51 gelangen kann, hat der Deckel nur an seinen Längsseiten nach oben vorspringende Randleisten 52, die außen Einschnitte 53 aufweisen, die komplementär mit ebenfalls nur längsseitig vorgesehenen unteren Randvorsprüngen 18 des Unterteils ausgebildet sind und als Abstandshalter und gleichzeitig als Positionierelemente beim Stapeln der Boxen aufeinander dienen. Damit ergibt sich, wie anhand der strichpunktiert angedeuteten aufgesetzten weiteren Box ersichtlich ist, ein von den Stirnenden der Boxen her offener und für den Sterilisierdampf zugänglicher Zwischenraum 55.

Für alle dargestellten Ausführungsformen gilt: Mindestens der Deckel 20, vorzugsweise aber auch das Unterteil 10 (bei dem Ausführungsbeispiel nach Fig. 8 umgekehrt) des Behältnisses ist aus einem transparenten, vorzugsweise glasklaren, thermostabilen, unzerbrechlichen, spülmaschinenfesten und spülmittelfesten Kunststoff gefertigt, der Temperaturen bis 140 °C, also den üblichen Sterilisiertemperaturen standhält. Beispiele solcher Kunststoffe sind Polysulfon und Polyäthersulfon.

Die Filterschicht besteht aus einem hydrophoben Filtergewebe mit einer Porendichtigkeit, die mindestens 0,5 $\mu$m beträgt oder noch besser ist.

Zu den Ausführungsbeispielen nach den Fig. 3 und 5 ist noch anzumerken, daß dort der Deckel als kleine Schale verwendet werden kann, und zwar der Deckel der Ausführungsform nach Fig. 3 in kopfstehendem Zustand (wie abgebildet), der Deckel nach Fig. 5 in seiner normalen Position, wodurch sonst übliche kleine Pappschalen oder dgl. entfallen können.

**Patentansprüche**

1. Sterilisierfähiges, mindestens teilweise durchsichtiges Behältnis zur Aufnahme einzelner medizinischer oder chirurgischer Bestecke, Instrumente oder Geräte zum Zwecke des Sterilisierens und anschließenden sterilen Aufbewahrens,

   dadurch gekennzeichnet, daß das Behältnis als mehrfach sterilisierfähige steife Box (10, 20 bzw. 10, 50) aus sterilisiertemperaturbeständigem Kunststoff ausgebildet ist, die aus einem Unterteil (10) und einem damit zusammenwirkenden und keimdicht abschließenden Deckel (20 bzw. 50) besteht und mittels einer keimdichten, jedoch für Sterilisierdampf bzw. Sterilisiergas durchlässigen Filterschicht (30) abgedeckte Wanddurchbrüche (11) aufweist.

2. Behältnis nach Anspruch 1, dadurch gekennzeichnet, daß es die Form einer flachen Box hat und die Wanddurchbrüche (11) in den Seitenwänden gebildet sind.

3. Behältnis nach Anspruch 1, dadurch gekennzeichnet, daß es eine etwa zylindrische Form mit becherförmigem Unterteil (10) und entsprechendem Deckel (20) hat und die Wanddurchbrüche (11) in der Umfangswand des becherförmigen Unterteils gebildet sind.

4. Behältnis nach Anspruch 2 oder 3, dadurch gekennzeichnet, daß die Filterschicht (30) zwischen den Unterteilwänden und einem in das Unterteil (10) eingesetzten komplementären Einsatz (40) sandwichartig gehaltert ist, wobei der Einsatz mit Wanddurchbrüchen (41) versehen ist, welche die Wanddurchbrüche des Unterteils (11) mindestens zum überwiegenden Teil überdecken.

5. Behältnis nach Anspruch 4, dadurch gekennzeichnet, daß die Seitenwände des Unterteils (10) und der Einsatz (40) jeweils sich nach oben erweiternd konisch ausgebildet sind und der Einsatz und die Unterteilwände in ihrem oberen Bereich mit miteinander zusammenwirkenden Rastelementen (43) versehen sind.

6. Behältnis nach Anspruch 2, dadurch gekennzeichnet, daß am Unterteilboden und am Deckel miteinander zusammenwirkende komplementäre Lagesicherungselemente (13, 23; 17; 18, 53) zum verschiebesicheren Aufeinanderstapeln mehrerer Behältnisse sowie seitliche Abstandhalter (14, 25) zur Sicherung gegenseitigen seitlichen Abstands bei nebeneinander gestapelten Behältnissen vorgesehen sind.

7. Behältnis nach Anspruch 2, dadurch gekennzeichnet, daß das Unterteil (10) sich nach oben erweiternd konisch verlaufende Seitenwände und der Deckel (20) einen nach oben hochgezogenen und den oberen Randteil (12) der Unterteilseitenwände umgreifend wieder nach unten umgefalzten Randwulst (25) aufweist und die Bemessung so abgestimmt ist, daß die zwischen dem Deckelhauptteil und dem Deckelrandwulst (25) gebildete flache Mulde (29) in ihrem Lichtmaß dem Außenmaß des Bodenbereichs des Unterteils (10) entspricht.

8. Behältnis nach Anspruch 7, dadurch gekennzeichnet, daß der Deckel (20) innenseitig einen nach unten vorspringenden, außen zum konischen Verlauf der Unterteilseitenwände komplementär geformten Steg (26) aufweist, der bei geschlossenem Deckel zusammen mit den Unterteilseitenwänden die Filterschicht (30) sandwichartig haltert und mit zu den Wanddurchbrüchen (11) der Unterteilseitenwände im wesentlichen deckungsgleich angeordneten Durchbrüchen (27) versehen ist.

**9.** Behältnis nach Anspruch 1, dadurch gekennzeichnet, daß es die Form einer flachen Box hat und die Wanddurchbrüche (51) im Deckel (50) gebildet sind, an dessen Innenseite die Filterschicht (30) mittels einer mit entsprechenden Durchbrüchen (61) versehenen Halteplatte (60) gehaltert ist.

**10.** Behältnis nach Anspruch 9, dadurch gekennzeichnet, daß am Bodenbereich des Unterteils (10) und an der Oberseite des Deckels (50) miteinander zusammenwirkende Lagesicherungs- und Abstandhalteelemente (18, 53) gebildet sind, die beim Aufeinanderstapeln mehrerer Behältnisse jeweils einen den Durchtritt von Sterilisierdampf bzw. Sterilisiergas ermöglichenden Zwischenraum (55) zwischen dem Boden und der Deckeloberseite zweier übereinandergestapelter Behältnisse sicherstellt.

**11.** Behältnis nach einem der Ansprüche 2, 3 oder 9, dadurch gekennzeichnet, daß das Unterteil (10) und der Deckel (20, 50) mit komplementär ineinandersteckbaren Profilen (12, 22) versehen sind, die im geschlossenen Zustand eine in Form mindestens einer vollständigen U-Schleife verlaufende Fuge ergeben.

**12.** Behältnis nach Anspruch 3, dadurch gekennzeichnet, daß das Unterteil (10) und der Deckel (20) miteinander einen Schraubverschluß bilden.

**13.** Behältnis nach einem der Ansprüche 1 bis 12, dadurch gekennzeichnet, daß die Filterschicht (30) als Band oder Streifen aus hydrophobem Filtergewebe ausgebildet ist.

**14.** Behältnis nach einem der Ansprüche 1 bis 13, dadurch gekennzeichnet, daß das gesamte Behältnis aus transparentem Kunststoff besteht.

**15.** Behältnis nach einem der Ansprüche 1 bis 14, dadurch gekennzeichnet, daß es aus einem Polysulfon oder einem Polyäthersulfon hergestellt ist.

EP 0 513 614 A1

FIG. 2

FIG. 1

8

FIG. 3

FIG. 4

FIG. 5

FIG. 6

FIG. 8

FIG. 7

Europäisches
Patentamt

**EUROPÄISCHER RECHERCHENBERICHT**

Nummer der Anmeldung

EP    92 10 7511

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X<br>A | US-A-3 503 497 (RIELY ET AL.)<br>* Spalte 5, Zeile 57 - Spalte 6, Zeile 4;<br>Ansprüche 1,7; Abbildung 4 *<br>* Spalte 8, Zeile 12 - Zeile 48 *<br>--- | 1<br>2,13,14 | A61C19/02<br>A61B19/02<br>A61L2/26 |
| X<br>A | DE-U-8 909 149 (ZL MICRODENT-ATTACHMENT GMBH)<br>* Seite 2, Absatz 3-4; Ansprüche 1,2;<br>Abbildungen *<br>--- | 1<br>3,14 | |
| A | DE-U-8 911 764 (HARTMANN)<br>* das ganze Dokument *<br>--- | 1,2 | |
| A | DE-C-865 640 (INGENIÖRSFIRMA VIGGO AB.)<br>* Anspruch 1; Abbildungen 1,6-8 *<br>--- | 1,4,5 | |
| A | FR-A-2 542 200 (FRANCE IMPLANT EST (S.A.R.L.))<br><br>* Abbildungen 1,2 *<br>--- | 1,6,7,9,<br>10 | |
| D,A | DE-U-8 711 391 (AESCULAP-WERKE AG)<br>* Seite 6, Zeile 19 - Zeile 20; Anspruch 1;<br>Abbildung 1 *<br>--- | 1,15 | **RECHERCHIERTE SACHGEBIETE (Int. Cl.5)** |
| A | DE-U-1 868 792 (LAUTENSCHLÄGER)<br>* Ansprüche 1-3,6; Abbildung *<br>--- | 1,4 | A61C<br>A61B<br>A61L |
| A | EP-A-0 266 688 (BASF AG)<br>* das ganze Dokument *<br><br>----- | 1,14,15 | |

Der vorliegende Recherchenbericht wurde für alle Patentansprüche erstellt

| Recherchenort | Abschlußdatum der Recherche | Prüfer |
|---|---|---|
| BERLIN | 09 JULI 1992 | KANAL P. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
..............................................................................
& : Mitglied der gleichen Patentfamilie, übereinstimmendes
    Dokument

EPO FORM 1503 03.82 (P0403)